# EUROPEAN PATENT APPLICATION

(11) **EP 2 343 061 A1**
(43) Date of publication of application: **13.07.2011**
(21) Application number: 09016051.6
(22) Date of filing: 28.12.2009
(51) Int. Cl.: A61K 9/50, A61K 36/00, A61K 36/15, A23L 2/00, A61K 8/00, A61P 17/00, A61P 35/00, A23L 1/30

(54) **Use of tall oil pitch extract and compositions which contain it**

(71) Applicant: Lipofoods, S.L., 08850 Gava (ES)
(72) Inventor: González Pons, Eulalia, 08921 Santa Coloma de Gramenet Barcelona (ES); Padró Escayola, David, 08014 Barcelona (ES); Depelley, Laurent, 56860 Séné (FR)
(74) Representative: Carvajal y Urquijo, Isabel

(57) **Abstract**

Use of the unsaponificable fraction of tall oil pitch in the preparation of a food, cosmetic and/or pharmaceutical composition for the treatment, care and/or prevention of a disease, disorder and/or condition associated with the 5-*α*-reductase activity.

## Description

### Field of the invention

The present invention relates to a tall oil pitch fraction composition for treatment, care and/or prevention of diseases, disorders and/or conditions associated with 5-*α-*reductase activity.

### Background of the invention

Testosterone is an androgen and the principal male sex hormone which is mainly produced by the testicular Leydig cells in varying amounts over a man life time. To a lesser extent, it is also secreted by the adrenal glands and adrenal cortex in both men and women. This hormone acts as a stimulant of the blood flow, the growth of certain tissues and secondary sex characteristics. It can also be prepared synthetically from other steroids, especially from cholesterol. The effects of this hormone become more evident during puberty when testosterone levels increase and the male body shows up physiological changes. Such changes include the development of male secondary sex characteristics like a deep voice, growth of beard, chest and armpits hair, increased oil production by the sebaceous glands, maturation of sex organs and sperm, adipose tissue distribution and increased libido.

Before testosterone joins to the intracellular receptors is often reduced to 5-*α-*dihydrotestosterone or DHT, which is an active-type male hormone, by the catalytic action of 5-*α*-reductase in the presence of nicotinamide adenine nucleotide phosphate (NADPH). Excessive production of DHT is the main cause of various androgen-related disorders such as prostatic cancer, benign prostatic hyperplasia (BPH), polycystic ovary syndrome, seborrhea, acne, female hirsutism, androgenic alopecia, and even nervous anorexia. Thus, decreased production of DHT by inhibiting 5-*α*-reductase is an ideal solution for treatment, care and/or prevention of conditions, disorders and/or conditions listed above.

There are two isoenzymes of 5-*α*-reductase in humans. One isoenzyme, 5-*α*-reductase type 1, predominates in the viscera and has been also located in the sebaceous glands and dermal papilla cells of human skin while the isoenzyme that mainly interacts within the prostatic tissues is designated as 5-*α*-reductase type 2. Both isoenzymes are hydrophobic and have an amino acid sequence analogy of approximately 50%.

Androgen dependent disorders such as acne vulgaris, androgenic alopecia and female hirsutism are characterized by an increased activity of 5-*α*-reductase type 1 in the skin target cells leading to an increase in local production of DHT.

The state of art describes different 5-*α*-reductase inhibitors for treatment, care and/or prevention of the androgen dependent disorders listed above. These compounds inhibit the action of 5-*α*-reductase and thus suppress the production of DHT in tissues, being currently used as agents for the treatment of benign prostatic hyperplasia, androgenic alopecia (WO 2006/099121, US 5578599), female hirsutism, seborrhea, acne and polycystic ovary syndrome (US 4377584).

This is the case of finasteride, which is used to treat enlarged prostate and risk of acute urinary retention. However, the administration of the approved formulation of finasteride has several and different side effects depending on the patient. For example, some adverse effects in men are sexual dysfunction/decreased or loss of libido, impotence, reduced volume of ejaculation, erectile dysfunction, or gynecomastia. Therefore it can not be managed and/or manipulated by pregnant women because it can produce malformations of the male fetus' external genitals.

Another compound such as dutasteride has also been introduced in drug therapies for symptomatic treatment of BPH and the risk of acute urinary retention, thus reducing the need of surgery like transurethral resection of the prostate and prostatectomy. Although dutasteride most common side effects are sex problems such as impotence and lack of sex desire, besides it can cause gynecomastia.

lsotretinoin is another 5-*α*-reductase inhibitor type 1, used as an anti-acne agent. Isotretinoin is an androgen mediator of the sebaceous glands and sebum production of the facial areas, scalp and areas not prone to acne, although its activity is significantly higher in sebaceous glands of the face and scalp compared to areas not prone to acne. It has been shown that during treatment with isotretinoin appears to be a significant reduction of testosterone and urinary excretion of androsterone, tetrahydrocortisone and tetrahydrocortisol. Currently, isotretinoin is being widely used to treat acne, however, it presents a series of side effects which make it unacceptable for different groups of people such as pregnant women or young women. Side effects include, for example teratogenicity, lip cheilitis, nasal epistaxis,, ocular conjunctivitis, dry skin, muscle pain, mental disorders, depression, aggressive tendencies or headaches.

Other 5-*α*-reductase inhibitors used to prevent hair loss, and for the treatment of female hirsutism and benign prostatic hyperplasia are tamsulosin and minoxidil. At recommended concentrations, these compounds have also side effects. Side effects of tamsulosin are dizziness, headache, tachycardia, arterial hypotension, rhinitis, constipation, diarrhea, nausea, vomiting, itching, hives, abnormal ejaculation and fatigue. As for minoxidil, the most frequently reported adverse reactions were dermatological in nature such as pruritus, dermatitis, dryness, skin irritation, eczema, skin exfoliation or hypertrichosis, as well as headaches, paresthesia, tachycardia, palpitations and hypotension.

Therefore, there is still a need in the state of the art to find new 5-*α*-reductase inhibitors which do not have or have fewer side effects than 5-*α*-reductase inhibitors mentioned stated above.

### Description of the Invention

The present invention provides a solution to the existing needs described earlier in the background of the invention. Surprisingly we found that the unsaponificable fraction of tall oil pitch inhibits 5-*α*-reductase. Hence, the aim of the present invention is the use of the unsaponificable fraction of tall oil pitch in the preparation of food, cosmetic and/or pharmaceutical compositions for the treatment, care and/or prevention of a disease, disorder and/or condition associated with the activity of 5-*α*-reductase.

In a preferred embodiment, the disease, disorder and/or condition associated with the activity of 5-*α*-reductase, is selected from the group consisting of benign prostatic hyperplasia, prostatic cancer, polycystic ovary syndrome, acne, hyperseborrhea, alopecia, hair loss and hirsutism.

In another particular embodiment, the activity of 5-*α*-reductase is expressed in the skin and/or scalp of mammals, preferably in humans.

In a particular embodiment, the food, cosmetic and/or pharmaceutical composition contains at least one alimentary, cosmetically and/or pharmaceutically acceptable excipient.

These compositions can be prepared by conventional methods known by the person skilled in the art ["Harry's Cosmeticology", Eight Edition (2000) Rieger MM, ed., New York Chemical Pub, NY, U.S.]

In another particular embodiment, the unsaponificable fraction of tall oil pitch in a food, cosmetic and/or pharmaceutical composition can also be incorporated into a food, cosmetic and/or pharmaceutical delivery system and/or sustained release system.

The term "delivery system" refers to a solvent, adjuvant, excipient or carrier through the unsaponificable fraction of tall oil pitch is administered. Such food, cosmetic or pharmaceutical carriers can be liquids such as water, oils or surfactants, including those of petroleum, animal, vegetable or synthetic origin. These are for example and without limiting sense peanut oil, soybean oil, mineral oil, sesame oil, castor oils, polysorbates, sorbitan esters, ether sulfates, sulfates, betaines, glycosides, maltosides, fatty alcohols, nonoxynols, poloxamers, polyoxyethylene, polyethylene glycols, dextrose, glycerol, digitonin and similars. In *"Remington's Pharmaceutical Sciences"* by E.W. Martin are described solvents, adjuvants or excipients as suitable carriers.

The term "sustained release" is used in a conventional sense referring to a delivery system through a compound is released gradually during a period of time and preferably, but not necessarily, with compound amounts being constant throughout the period of time of the release.

Examples of delivery systems or sustained release systems are liposomes, mixed liposomes, oleosomes, niosomes, ethosomes, milliparticles, microparticles, miniparticles, nanoparticles and lipid solid nanoparticles, nanostructured lipid carriers, sponges, cyclodextrins, vesicles, micelles, mixed micelles of surfactants, mixed micelles of phospholipid-surfactant, millispheres, microspheres and nanospheres, lipospheres, millicapsules, microcapsules and nanocapsules, and microemulsions, nanoemulsions which can be added for greater penetration of the unsaponificable fraction of tall oil pitch and/or improve bioavailability and/or water solubility thereof. In cases where vehicle systems or sustained release permit it, a micronization can be made in order to reduce their size and thus improve the organoleptic properties of food, cosmetic and/or pharmaceutical compositions.

Sustained release systems can be prepared by methods known in the state of the art, and compositions containing them can be administered, for example, by topical administration, including adhesive patches, non-adhesive patches and microelectronic patches, or systemic administration, such as for example and without limiting sense, by oral or parenteral administration, including nasal, rectal, subcutaneous implantation or injection, or implantation or direct injection into a specific part of the body, and preferably with a gradual constant release of the unsaponificable fraction of tall oil pitch. The amount of unsaponificable fraction of tall oil pitch contained in the sustained release system depends on, for example, the site of administration, the kinetics and release time of the unsaponificable fraction of tall oil pitch and the nature of the condition, disorder and/or disease to be treated or prevented.

Moreover, the quantity of unsaponificable fraction of tall oil pitch of the food, cosmetic and/or pharmaceutical compositions is between 0.2% and 99% of the total weight of the composition, preferably between 20% and 95% of the total weight of the composition if it is an intermediate composition, and preferably between 0.2% and 70% of the total weight of the composition if it is a final composition.

Likewise, food, cosmetic and/or pharmaceutical compositions containing the unsaponificable fraction of tall oil pitch can be used in different types of formulations for oral administration, preferably in the form of food, oral cosmetics or oral drugs, as for example and without limiting sense in capsules, including gelatin capsules, tablets, including sugar coated tablets, powders, granules, chewing gum, solutions, suspensions, emulsions, syrups, polysaccharide films, jams and jellies, as well as any other formulation known by a person skilled in the art. In particular, the unsaponificable fraction of tall oil pitch can be incorporated into any form of functional food or fortified food, as for example and without limiting sense, dietary bars or compact or non-compact powders. These powders can be solubilised in water, soda, dairy products, cereal products, soy products, food supplements or incorporated into dietary bars. Unsaponifiable fraction of tall oil pitch may be formulated with common excipients and adjuvants for oral compositions or food supplements, such as for example and without limiting sense fat components, aqueous components, humectants, preservatives, texturizing agents, flavors, aromas, antioxidants and common colours in the food sector.

This food, cosmetic and/or pharmaceutical composition comprises at least one alimentary, cosmetically or pharmaceutically acceptable excipient or adjuvant, selected without limiting sense from the group of other 5-*α*-reductase inhibitor agents, anti-inflammatory agents and/or analgesic agents, extracts or combination of extracts that retard hair loss and/or a retardant compound of hair loss or hair growth stimulating agents, inhibitor agents of body hair growth, inhibitors of matrix metalloproteinases, antioxidants, agents stimulating wound healing, healing coadjuvant agents and/or reepithelialization agents, agents stimulating synthesis of keratin, agents stimulating proliferation of keratinocytes, keratolytic agents, exfoliating agents, desquamating agents, comedolytic agents, *α*-hydroxy acids, *β*-hydroxy acids, skin conditioners, solvents, moisturizers, vitamins, pigments or colourings, dyes, gelling polymers, thickeners, surfactants, agents regulating sebum production, lipolytic agents or stimulators of lipolysis, antipruritic agents, agents for the treatment or care of sensitive skin, antimicrobial agents, fungicidal agents, fungistatic agents, bactericidal agents, bacteriostatic agents, perfumes, chelating agents, plant extracts, essential oils, marine extracts, agents from biofermentation process, minerals, and cell extracts among others, provided they are physically and chemically compatible with the other components of the composition and especially with the unsaponificable fraction of tall oil pitch. Likewise, the nature of these additional ingredients should not unacceptably alter the benefits of the unsaponificable fraction of tall oil pitch. The nature of these additional ingredients can be from synthetic or natural origin, such as plant extracts, or obtained from biofermentation process. Additional examples can be found described in CTFA International Cosmetic Ingredient Dictionary & Handbook, 12th Edition (2008).

A particular embodiment of the present invention relates to a food, cosmetic and/or pharmaceutical composition which includes a cosmetically and/or pharmaceutically effective quantity of unsaponificable fraction of tall oil pitch and a quantity of at least one extract with 5-*α*-reductase inhibitory activity. The extract is selected for example and without limiting sense, from the group formed *by Cinnamommum zeylanicum extract, Laminaria saccharina, Spiraea ulmaria, Nettle Root, Pygeum africanum, Avena Sativa, Serenoa repens,* plant extracts *Arnica montana, Cinchona succirubra, Eugenia caryophyllata, Humulus lupulus, Hypericum perforatum, Mentha piperita, Rosmarinus officinalis, Salvia officinalis, Thymus vulgaricus,* plant extracts of the genus *Silybum,* plant extracts containing sapogenins and particularly Dioscorea plant extract among others and/or a cosmetic or pharmaceutical effective quantity for at least a synthetic compound, extract or product from a biofermentation process with 5-*α*-reductase inhibitory activity as for example and without limiting sense retinoids and in particular retinol, sulphur and its derivatives, zinc salts and in particular lactate, gluconate, pidolate, carboxylate, zinc salicylate or cysteate, selenium chloride, vitamin B6, pyridoxine, capryloyl glycine, sarcosine, finasteride, dutasteride, isotretinoin, tamsulosin and minoxidil among others.

In another particular embodiment, the food, cosmetic and/or pharmaceutical composition comprises at least one anti-inflammatory agent and/or analgesic agent selected from the group consisting for example and without limiting sense, mometasone furoate and prednisolone, anti-inflammatories, including steroidal and non-steroidal cyclooxygenase or lipoxygenase inhibitors, benzydamine, acetylsalicylic acid, rosmarinic acid, ursolic acid, glycyrrhizinate derivatives, *α*-bisabolol, azulene and analogues, sericoside, ruscogenine, escin, escoline, rutin and analogues, hydrocortisone, its salts and niflumic acid derivative; retinoids as for example and without limiting sense, t-trans-retinoic acid or tretinoin, isotretinoin, retinol or vitamin A and its derivatives such as acetate, palmitate, propionate, motretinide, etretinate and zinc transretinoate, clobetasol, dexamethasone, prednisone, paracetamol, acetylsalicylic acid, amoxiprin, benorylate, choline salicylate, diflunisal, faislamine, methyl salicylate, magnesium salicylate, salsalate, diclofenac, aceclofenac, acemetacin, bromfenac, etodolac, indometacin, sulindac, tolmetin, ibuprofen, dexiprofeno, carprofen, fenbufen, fenoprofen, flurbiprofen, ketoprofen, dexketoprofene, ketorolac, loxoprofen, naproxen, miroprofen, oxaprozin, pranoprofen, tiaprofenic acid, suprofen, mefenamic acid, meclofenamate, meclofenamic acid, fluphenamic acid, tolfenamic acid, nabumetone, phenylbutazone, azapropazone, clofezone, kebuzone, metamizole, mofebutazone, oxyphenbutazone, sulfinpyrazone, piroxicam, lomoxicam, meloxicam, tenoxicam, celecoxib, etoricoxib, lumiracoxib, parecoxib, rofecoxib, valdecoxib, nimesulide, naproxcinod, fluproquazone, licofelone, fatty acids omega-3 and omega-6, morphine, codeine, oxycodone, hydrocodone, diamorphine, pethidine, tramadol, buprenorphine, benzocaine, lidocaine, chloroprocaine, tetracaine, procaine, tricyclic antidepressants, amitriptyline, carbamazepine, gabapentine, pregabaline, panthenol, biotin, disodium lauriminodipropionate tocopheryl phosphate, ciclopirox olamine, nordihydroguaiaretic acid, Neutrazen™ [INCI: Water (Aqua), Butylene Glycol, Dextran, Palmitoyl Tetrapeptide-8] marketed by Atrium Innovations/Unipex Group, Meliprene^{®} [INCI: Dextran, Acetyl Heptapeptide-1] marketed by Institut Européen de Biologie Cellulaire/Unipex Group, coenzyme Q10 or alkylglycerine ethers, and/or mixtures thereof, or natural extracts or essential oils with intrinsic analgesic and/or anti-inflammatory activity, such as, including but not limited to, madecassoside, echinacea, amaranth seed oil, sandalwood oil, peach tree leaf extract, *Aloe vera, Arnica montana, Artemisia vulgaris, Asarum maximum, Calendula officinalis, Capsicum, Centipeda cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Origanum vulgare, Prunus laurocerasus, Thymus vulgaris, Salix alba, Silybum marianum, Tanacetum parthenium, Uncaria guianensis or Vaccinum myrtillus* extract and/or mixtures thereof, among others.

In another particular embodiment, the food, cosmetic and/or pharmaceutical composition comprises at least one extract or combination of extracts that retard hair loss or stimulate hair growth selected from the group consisting for example and without limiting sense, *Tussilago farfara* or *Achillea millefolium, Jaborandi, Urtica dioica, Swertia japonica, Catha edilus, Celosia argentea, Assian Radix,* and *Rosmarinus officinalis,* and/or also a cosmetically or pharmaceutically effective amount of at least one retardant hair loss compound or hair growth stimulating agent selected from the group consisting of for example and without limiting sense nicotinic acid esters such as C3-C6 alkyl nicotinate such as methyl or hexyl nicotinate, benzyl nicotinate or tocopheryl nicotinate, benzalkonium chloride, benzethonium chloride, phenol, estradiol, histamine antagonists such as diphenhydramine hydrochloride, chlorpheniramine maleate, chlorophyllin derivatives, cholesterol, salicylic acid, cysteine, methionine, red pepper tincture, benzyl nicotinate, menthol, peppermint oil, calcium pantothenate, panthenol, castor oil, hinokitiol, prednisolone, resorcinol, monosaccharides and esterified monosaccharides, chemical activators of protein kinase C enzymes, glycosaminoglycan chain cellular uptake inhibitors, inhibitors of glycosidase activity, glycosaminoglycanase inhibitors, esters of pyroglutamic acid, hexasaccharic acids or derivatives thereof, glucosaccharic acid, aryl-substituted ethylenes, N-acylated amino acids, cyclosporins, and cyclosporin analogues such as cyclosporin A, potassium channel blockers, such as minoxidil, androgen receptor antagonists, such as cyproterone acetate, retinoic acid, dexpenthenol, *α*-hydroxy acids as for example benzophenone, and anticonvulsants of hydantoin and phenytoin; cyclosporin and cyclosporin analogs, tacrolimus, thyroid hormones, prostaglandin agonists or antagonists, retinoids as for example acitretin and tazarotene; flavinoids and analogs, ascomycin derivatives and analogs, triterpenes such as oleanolic acid and ursolic acid; proteoglycanase or glycosaminoglycanase inhibitors, estrogen agonists and antagonists, pseudoterins, cytokine and growth factor promotors analogs or inhibitors such as interleukinil inhibitors, interleukin-6 inhibitors, interleukin-10 promotors, and vitamins such as vitamin D analogs; parathyroid hormone antagonists, vitamin B12 analogs, interferon agonists and antagonists, vasodilators such as sodium lauroyl, cysteine and lysine chlorohydrate; antibacterial agents, such as, but not limited to macrolides, piranosides, erythromycin and tetracyclines; calcium channel antagonists, such as, but not limited to, cinnarizine and diltiazem; hormones, such as, but not limited to estril, their analogues or thyroxine and/or their salts; antiandrogenic agents, such as, but not limited to oxendolone, spironolactone or diethylstilbestrol; scavenging agents, such as, but not limited to dimethyl sulfoxide; esterified oligosaccharides; glucosidase inhibitors, such as, but not limited to D-glycaro-1,5-lactam; glycosaminoglycanase and proteoglycanase inhibitors, such as, but not limited to L-galactono-1,4-lactone; tyrosine kinase inhibitors, such as, but not limited to 1-amino-1-cyano(3,4-dihydroxyphenyl)ethylene, diazoxide, such as, but not limited to 7-(acetylthio)-4',5'-dihydrospiro[androst-4-ene-17,2'-(3H)furan]-3-one, 1,1-dioxide 3-methyl-7-chloro[2H]-1,2,4-benzothiadiazine or spiroxazone; hydroxy- or ketocarboxylic acids and esters thereof, lactones and their salts; anthralin, eicosa-5,8,11-triinoic acid and esters or amides or minoxidil and its derivatives among others.

In another particular embodiment, the food, cosmetic and/or pharmaceutical composition comprises at least one antioxidant selected from the group consisting for example and without limiting sense, butylhydroxyanisole (BHA), 2,6,-di-tert-butyl-4 methylphenol (BHT), propyl gallate, probucol, sulfite, thiols, polyphenols, ascorbic acid or salts thereof, citric acids, citrates, monoglyceride esters, disodium edetate, lactic acid, malic acid, succinic acid, tartaric acid , vitamin A or *β*-carotene, vitamins E and C, copper, mannitol, tocopherol, reduced glutathione, carotenoids such as cryptoxanthin, astaxanthin and lycopene; cysteine, uric acid, taurine, tyrosine, superoxide dismutase, lutein, zeaxanthin, N-acetyl-cysteine, carnosine, gamma-glutamylcysteine, quercetin, lactoferrin, dihydrolipoic acid, *Ginkgo Biloba* extract, tea catechins, bilberry extract, retinyl palmitate, and its derivatives, bisulfate, metabisulfites, lecithin, chromanes, chromenes and analogues, lipochroman, metal chelating agents, such as ethylenediamine tetraacetic acid, sorbitol, tartaric acid and phosphoric acid; herbal extracts, such as rosemary, sage, ginger, marjoram, green tea or black tea extracts; rosemary oleoresin extract, plant extracts containing phenols such as vanillin, ellagic acid and resveratrol; and synthetic antioxidants, such as tertiary butylhydroquinone or mixtures thereof, metal salts such as selenium, cadmium, zinc or vanadium, particularly metals with a +2 valence; other compounds such as repaglinide, carnosine, coenzyme Q, idebenone or its derivatives.

In another particular embodiment, the food, cosmetic and/or pharmaceutical composition comprises at least one agent stimulating healing, a healing coadjuvant agent and/or reepithelialization agent selected from the group consisting for example and without limiting sense, extracts of *Aristoloquia clematis, Centella asiatica, Rosa moschata, Echinacea angustifolia, Symphytum officinale, Equisetum arvense, Hypericum perforatum, Mimosa fenuiflora, Persea gratisima, Prunus africanum, Tormentilla erecta, Aloe vera,* Polyplant^{®} Epithelizing [INCI: Calendula Officinalis, Hypericum Perforatum, Chamomilla Recutita, Rosmarinus Officinalis] marketed by Provital, Cytokinol^{®} LS 9028 [INCI: Hydrolyzed Casein, Hydrolyzed Yeast Protein, Lysine HCI] marketed by Laboratories Serobiologiques/Cognis o Deliner^{®} [INCI: Zea May (Corn) Kernel Extract] marketed by Coletica/Engelhard among others, and/or additionally, a cosmetically or dermopharmaceutically effective amount of at least one synthetic compound, extract or product coming from a biofermentation process with healing and/or reepithelizing activity or with effectiveness as coadjuvant in processes of healing and/or reepithelizing such as for example and in a non-limiting sense, allantoin, cadherins, integrins, selectins, yeast, panthenol, hexylresorcinol, phenol, tetracycline chlorhydrate, laminin and keratin; hyaluronic acid receptors, immunoglobulins, fibroblasts growth factor, connective tissue growth factor, platelet derived growth factor, vascular endothelial growth factor, epidermal growth factor, insulin-like growth factor, keratinocyte growth factor, colony-stimulating factors, transforming growth factor-beta, tumor necrosis factor-alpha, interferons, interleukins, matrix metalloproteinases, protein tyrosine phosphatase receptors, Antarcticine^{®} [INCI: Pseudoalteromones Ferment Extract] or Decorinyl^{®} [INCI: Tripeptide-10 Citrulline], marketed by Lipotec, among others.

In another particular embodiment, the food, cosmetic and/or pharmaceutical composition comprises at least one agent stimulating the synthesis of keratin selected from the group consisting for example and without limiting sense, by products derived from the glycoprotein extracts of *Klebsiella pneumoniae* strain, biochinone, pantolactone, minoxidil, L-omithine, allyl isocianate, horseradish extract, rhodanide ions, copper (II) ions, sulphur-containing amino acids or mixtures thereof such as cysteine and methionine; 2-thioacetamide, *Gotu kola,* carotene or provitamin A, alkanolamine such as ethylaminoethanol, methylaminoethanol, dimethylaminoethanol, isopropanolamine, triethanolamine, isopropanoldimethylamine, ethylethanolamine, 2-butanolamine, choline, serine, and mixtures thereof.

In another particular embodiment, the food, cosmetic and/or pharmaceutical composition comprises at least one keratolytic agent and/or exfoliating agent and/or desquamating agent selected from the group consisting for example and without limiting sense, hydroxy acids and their derivatives, *β*-hydroxy acids, in particular salicylic acid and its derivatives, or gentisic acid; *α*-hydroxy acids and their salts, such as glycolic acid, ammonium glycolate, lactic acid, 2-hydroxyoctanoic acid, *α-*hydroxycaprylic acid, mandelic acid, citric acid, malic acid or tartaric acid, *α*- and *β-*hydroxybutyric acid, polyhydroxy acids such as gluconic acid, glucuronic acid or sacaric acid; keto acids such as pyruvic acid, glyoxylic acid; pyrrolidonecarboxylic acid, cysteic acid and derivatives, aldobionic acids, azelaic acid and its derivatives as the azeloyl diglycinate; ascorbic acid and its derivatives, such as ascorbyl phosphate, ascorbyl glucoside and sodium or magnesium ascorbyl phosphate, nicotinic acid and its esters, and nicotinamide or vitamin B3, nordihydroguaiaretic acid, urea, oligofucoses, cinnamic acid, jasmonic acid derivatives, hydroxystilbenes such as resveratrol; *Saccarum officinarum* extract, enzymes involved in desquamation or degradation of comeodesmosomes such as glycosidases, stratum corneum chymotryptic enzyme (SCCE) or other proteases such as trypsin, chymotrypsin, sutilains, papain or bromelain, chelating agents such as EDTA, aminosulfonic compounds such as 4-(2-hydroxyethyl)piperazine-1-ethanesulfonic acid (HEPES) or sodium methylglycine diacetate (TRILON^{®} M marketed by BASF); derivatives of 2-oxothiazolidine-4-carboxylic acid (procysteine); sugar derivatives such as O-octanoyl-6-D-maltose and *N*-acetylglucosamine; chestnut extract (*Castanea sativa*) as the one marketed by SILAB under the name of Recoverin^{®} [lNCl: Water (Aqua), Castanea Sativa Seed Extract]; opuntia extract (*Opuntia ficus-indica*) as marketed by SILAB as Exfolactive^{®} [INCI: Opuntia Ficus lndica Flower Extract hydrolyzed]; or Phytosphingosine SLC^{®} [INCI: Salicyloyl Phytosphingosine] marketed by Degussa/Evonik, extract or combination of extracts of *Saphora japonica,* papaya, pineapple, pumpkin, sweet potato and/or mixtures thereof. Preferably, desquamating agents and/or exfoliating agents and/or keratolytic agents are selected from the group consisting of salicylic acid, ammonium glycolate, lactic acid, malic acid, citric acid, urea, resveratrol, EDTA, papain, bromelain, or an extract or combination of extracts of *Saphora japonica ,* papaya or pineapple and/or mixtures thereof. More preferably, are selected from the group consisting of urea and EDTA.

A further aspect of the present invention relates to a food, cosmetic or pharmaceutical method for the treatment, care and/or prevention of those diseases, disorders and/or conditions that result from the activity of 5-*α-*reductase, which comprises the administration of an effective amount of unsaponificable fraction of tall oil pitch, preferably in the form of a food, cosmetic and pharmaceutical composition. The present invention also provides a food, cosmetic or pharmaceutical method to inhibit the enzyme 5-*α*-reductase.

### Examples

### General Methodology

All reagents and solvents were of quality for synthesis and were used without further treatment.

### Example 1

### Evaluation of the inhibitory activity of the unsaponificable fraction of tall oil pitch on the activity of 5-α-reductase by measuring the rate of dihydrotestosterone formed from testosterone throughout rat liver microsomes

Firstly, the rat liver hepatic microsomes were obtained from a group of Sprague-Dawley (Becton Dickinson). Testosterone and nicotinamide adenine dinucleotide phosphate (NADPH) were obtained from Sigma. The plates for the test were obtained from Greiner.

Then, a pattern of microsomes was prepared and they were used at a final concentration of 0.5 mg/ml which were reacted with testosterone. The reaction substrate, testosterone, was used at a final concentration of 40 *µ*mol/L. The inhibitor of 5-*α*-reductase, the unsaponificable fraction of tall oil pitch was prepared at a final concentration of 20 *µ*mol/L. NADPH was added to a final concentration of 88 *µ*mol/L. The reaction took place in a buffer consisting of tris(hydroxymethyl)aminomethane (Tris), EDTA-Na₂, NaCl, sucrose, HCl 1N, dimethyl sulfoxide (DMSO) and MgCl₂ 6H₂O.

Once all the reagents were prepared, the inhibitory activity of the enzyme 5-*α-*reductase in the hepatic microsomes was determined and the results are summarized in Table 1. This was assessed through the incubation of microsomes for 10 minutes with absorbance readings at 340 nm every 30 seconds. The reaction was carried out at a temperature of 37 °C. The trial was carried out in a 96 well plate and samples were performed in triplicate. In each experiment the absorbance of the blank (buffer, microsomes and NADPH), negative control (buffer, microsomes, NADPH and testosterone), and test sample (buffer, microsomes, NADPH, and unsaponificable fraction of tall oil pitch) was measured.

| **Table 1. Evaluation of the 5-α-reductase inhibitory activity in liver microsomes throughout the action of the unsaponificable fraction of tall oil pitch.** | | |
|---|---|---|
| Sample | % Activity 5-*α*-reductase | % inhibition 5-*α*-reductase |
| Negative control | 100 | 0 |
| Test sample | 74.97 | 25.03 |

### Example 2

### Microcapsules containing unsaponificable fraction of tall oil pitch.

The microcapsules were prepared in dispersible form from unsaponificable fraction of tall oil pitch to which maltodextrin, inulin and sucrose esters were added.

First, 69.2 g of water dissolved 1.2 g of sucrose esters (an emulsifier), 5.9 g of maltodextrin and 1.2 g of inulin (encapsulants) and 22.5 unsaponificable fraction of tall oil pitch.

Then the product was milled to reduce particle size. Finally, the dispersion (atomization) was dried to produce a microcapsule powder with a composition of 19.2% maltodextrin, 3.9% inulin, 3.9% sucrose ester and 73% unsaponificable fraction of tall oil pitch.

### Example3

### Microcapsules containing unsaponificable fraction of tall oil pitch.

Microcapsules were prepared from unsaponificable fraction of tall oil pitch under the same procedure reported for example 3 but without inulin in order to obtain a powder of microcapsules with a final composition of 4.55% maltodextrin, 3.75% sucrose ester and 91.7% of unsaponificable fraction of tall oil pitch.

### Example 4

### Preparation of a yoghurt fortified with unsaponificable fraction of tall oil pitch.

The microcapsules of example 3 were taken and the microcapsule was added in liquid form, prepared from water, unsaponificable fraction of tall oil pitch, maltodextrin, sucrose ester, xanthan gum and citric acid and which it contained an 87% of unsaponificable fraction of tall oil pitch. The liquid form was used in the production of fruit preparations for yoghurt.

Finally, the yoghurt was pasteurized at 100°C for 4 minutes and aseptically packaged.

### Example 5

### Preparation of juice fortified with unsaponificable fraction of tall oil pitch.

The microcapsules from example 2 were taken and the microcapsule in liquid form, prepared from water unsaponificable fraction of tall oil pitch, maltodextrin, inulin, sucrose ester, xanthan gum and citric acid, was then used in the preparation of fortified juices containing 18% unsaponificable fraction of tall oil pitch.

### Example 6.

### Composition of unsaponificable fraction of tall oil pitch.

A composition containing unsaponificable fraction of tall oil pitch (18% by weight); sulphur containing amino acids (7%); zinc (0.5% by weight); *Aloe Vera* extract (5%) and water q.s.p.100% by weight was prepared.

### Example 7

### Composition of unsaponificable fraction of tall oil pitch.

A composition containing unsaponificable fraction of tall oil pitch (29% by weight); sulphur containing amino acids (11%); zinc (0.3% by weight); ursolic acid (25%) and water q.s.p. 100 % by weight was prepared.

### Example 8

### Composition of unsaponificable fraction of tall oil pitch.

A composition containing unsaponificable fraction of tall oil pitch (0.9% by weight); sulphur containing amino acids (3%); zinc (0.1 wt%); rosmarinic acid (21%); retinol (3 %); *Hypericum perforatum* extract (2%); *Rosmarinus officinalis* extract (3.8%) and olive oil to complete 100% by weight was prepared.

### Example 9

### Composition of unsaponificable fraction of tall oil pitch.

A composition containing unsaponificable tall oil pitch extract (5% by weight); sulphur containing amino acids (0.8%); zinc (1.6% by weight); vitamin B (0.3%); lycopene (2%) and water q.s.p. 100% by weight was prepared.

## Claims

1. Use of the unsaponificable fraction of tall oil pitch in the preparation of a food, cosmetic and/or pharmaceutical composition for the treatment, care and/or prevention of a disease, disorder and/or condition associated with the 5-*α*-reductase activity.

2. Use according to claim 1, wherein the disease, disorder and/or condition associated with the 5-*α*-reductase activity is selected from the group consisting of benign prostatic hyperplasia, prostate cancer, polycystic ovary syndrome, acne, hyperseborrhea, alopecia, hair loss and hirsutism.

3. Use according to any of claims 1 to 2, where the activity of 5*α*-reductase is expressed in the skin and/or scalp of mammals.

4. Use according to claim 1, wherein the unsaponificable fraction of tall oil pitch is incorporated into a delivery system and/or sustained release system selected from the group consisting of liposomes, mixed liposomes, oleosomes, niosomes, ethosomas, milliparticles, microparticles, miniparticles , nanoparticles, solid lipid nanoparticles, nanostructured lipid carriers, sponges, cyclodextrins, vesicles, micelles, mixed micelles of surfactants, surfactant-phospholipid mixed micelles, millispheres, microspheres, nanospheres, lipospheres, milicapsules, microcapsules, nanocapsules, microemulsions and nanoemulsions.

5. Use according to claim 1, wherein the composition is administered orally, topically and/or parenterally.

6. Use according to claim 1, wherein the amount of unsaponificable fraction of tall oil pitch is between 0.2% and 99% of the total weight of the composition.

7. Use according to claim 1, wherein the composition comprises at least one alimentary, cosmetically or pharmaceutically acceptable excipient or adjuvant.

8. Use according to claim 7, where the alimentary, cosmetically or pharmaceutically acceptable excipient or adjuvant is selected from the group of other 5-*α*-reductase inhibitor agents, anti-inflammatory agents and/or analgesic agents, extracts or combination of extracts that retard hair loss and/or a retardant compound of hair loss or hair growth stimulating agents, inhibitor agents of body hair growth, inhibitors of matrix metalloproteinases, antioxidants, agents stimulating wound healing, healing coadjuvant agents and/or reepithelialization agents, agents stimulating synthesis of keratin, agents stimulating proliferation of keratinocytes, keratolytic agents, exfoliating agents, desquamating agents, comedolytic agents, *α*-hydroxy acids, *β*-hydroxy acids, skin conditioners, solvents, moisturizers, vitamins, pigments or colourings, dyes, gelling polymers, thickeners, surfactants, agents regulating sebum production, lipolytic agents or stimulators of lipolysis, antipruritic agents, agents for the treatment or care of sensitive skin, antimicrobial agents, fungicidal agents, fungistatic agents, bactericidal agents, bacteriostatic agents, perfumes, chelating agents, plant extracts, essential oils, marine extracts, agents from biofermentation process, minerals, and cell extracts.
